# EUROPEAN PATENT SPECIFICATION

(11) **EP 2 046 745 B1**
(45) Date of publication and mention of the grant of the patent: **23.10.2013**
(21) Application number: 07788151.4
(22) Date of filing: 01.08.2007
(51) Int. Cl.: C07D 211/90

(54) **PROCESS FOR PREPARING AMORPHOUS LERCANIDIPINE HYDROCHLORIDE**
VERFAHREN ZUR HERSTELLUNG VON AMORPHEM LERCANIDIPINHYDROCHLORID
PROCÉDÉ DE PRÉPARATION DU CHLORHYDRATE DE LERCANIDIPINE AMORPHE

(30) Priority: 04.08.2006 IT MI20061576; 04.08.2006 IT MI20061577
(43) Date of publication of application: 15.04.2009
(73) Proprietor: Recordati Ireland Limited, Ringaskiddy County Cork (IE)
(72) Inventor: LEONARDI, Amedeo, 20154 Milano (IT); MOTTA, Gianni, 20030 Barlassina (Milano) (IT); JACQUET, Luc, 21047 Saronno (Varese) (IT)
(74) Representative: Pistolesi, Roberto
(86) International application number: PCT/EP2007/057986
(87) International publication number: WO 2008/015248

(56) References cited:
- EP-A1- 1 860 102
- WO-A-2006/089787
- US-A- 4 705 797

## Description

The present invention provides a process for preparing substantially pure amorphous lercanidipine hydrochloride.

### BACKGROUND OF THE INVENTION

Lercanidipine (methyl 1,1,N-trimethyl-N-(3,3-diphenylpropyl)-2-aminoethyl 1,4-dihydro-2,6-dimethyl-4-(3-nitrophenyl)pyridine-3,5-dicarboxylate) is a highly lipophilic dihydropyridine calcium antagonist with a long duration of action and high vascular selectivity. Lercanidipine's biological activity derives from its ability to competitively antagonize the dihydropyridine subunit of the L-type calcium channel.

Lercanidipine is useful as an anti-hypertensive. Lercanidipine lowers blood pressure by blocking calcium channels of arterial smooth muscle, thus decreasing peripheral vascular resistance. Lercanidipine produces no negative cardiac inotropism and only occasional mild reflex tachycardia, which is generally of short duration. Lercanidipine has been approved for the treatment of hypertension and has been marketed since 1996 in several European countries under the trademark Zanidip^{™}.

The hydrochloride salt of lercanidipine is commercially available from Recordati S.p.A. (Milan, Italy). Methods of preparing lercanidipine hydrochloride, as well as methods of resolving lercanidipine into individual enantiomers are described in US 4705797, US 5767136, US 4968832, US 5912351, US 5696139, US 2003/0069285 and US 2003/0083355.

US 4705797 described a process for the preparation of lercanidipine, the final step of the process being a cyclisation between 1,1,N-trimethyl-N-(3,3-diphenylpropyl)-2-aminoethyl α-acetyl-3-nitrocinnammate and methyl 3-aminocrotonate. Lercanidipine was isolated as its hydrochloride by crystallization from water containing HCl and NaCl. However, this process was expensive, time consuming, and resulted in relatively low yields of lercanidipine hydrochloride. The product was a crude ill-defined mixture, mainly of amorphous lercanidipine hydrochloride but possibly containing from 1% to 2% of crystalline lercanidipine hydrochloride. The product had a hydration ratio of from about 0.3:1 to 0.5:1, and contained less than 95% of lercanidipine hydrochloride (crystalline form included).

Such a product is too impure for use in pharmaceutical compositions, and would require extensive further purification, e.g., by chromatography on different phases, before it would be suitable for such use. However, purification by these methods is too costly and time consuming for commercial application.

WO 2006/046830 discloses that amorphous lercanidipine has an improved solubility and bioavailability and may be obtained starting from crystalline lercanidipine.

PCT/EP2006/001782, filed on February 24, 2006, claiming a US priority of February 25, 2005, discloses amorphous lercanidipine hydrochloride and methods for preparing the same. Amorphous lercanidipine hydrochloride is well suited for incorporation into pharmaceutical compositions and solid dosage forms, particularly modified release pharmaceutical dosage forms comprising a waxy matrix as a release modifying agent. Amorphous lercanidipine hydrochloride may also be advantageously incorporated into immediate release pharmaceutical compositions that have improved pharmacokinetic properties and consequently provide rapid reduction in hypertension when administered to a patient. Amorphous lercanidipine hydrochloride begins exerting its activity to reduce blood pressure within a period of time following its administration that is markedly shorter than the time required for obtaining an effect following administration of crystalline lercanidipine hydrochloride.

### SUMMARY OF THE INVENTION

The invention provides a process for preparing substantially pure amorphous lercanidipine hydrochloride by precipitation. The amorphous lercanidipine hydrochloride prepared as disclosed herein is substantially pure and has a greater aqueous solubility and faster onset of the antihypertensive effect when administered to a patient, compared to crystalline lercanidipine hydrochloride.

In particular, the invention provides a method of preparing amorphous lercanidipine hydrochloride comprising (a) dissolving Lercanidipine in an organic solvent to form a first solution, (b) contacting such a first solution with a second water and/or organic HCl solution, (c) optionally adding water and, (d) recovering the precipitated amorphous lercanidipine hydrochloride.

### DETAINED DESCRIPTION OF THE INVENTION

As used herein, the following terms are defined as follows:

The term "lercanidipine" refers to methyl 1,1,N-trimethyl-N-(3,3-diphenylpropyl)-2-aminoethyl 1,4-dihydro-2,6-dimethyl-4-(3-nitrophenyl)-pyridine-3,5-dicarboxylate, both under free base and salified form. The term "lercanidipine hydrochloride" refers to the hydrochloride salt of methyl 1,1,N-trimethyl-N-(3,3-diphenylpropyl)-2-aminoethyl 1,4-dihydro-2,6-dimethyl-4-(3-nitraphenyl)-pyridine-3,5-dicarboxylate. Lercanidipine salt may be present as one or both of its enantiomeric forms.

The term "amorphous" refers to solid compounds having no substantial crystal lattice structure. Amorphous compounds typically yield DSC plots with broad endothermic transitions, defined as glass transitions. Crystalline compounds, by comparison, typically exhibit shap exothermic peaks.

As used herein, the "substantially pure" refers to a composition that is at least 95% pure, preferably at least at least about 97% pure, more preferably at least about 99% pure and still more preferably at least about 99.5% pure on a weight/weight basis relative to contaminants, including solvents carried over from the preparation of the composition.

The invention provides a process for preparing amorphous lercanidipine hydrochloride. The process disclosed herein yield amorphous lercanidipine hydrochloride in a substantially pure state, having improved solubility and different pharmacokinetics properties compared to those of other known forms of lercanidipine hydrochloride The amorphous lercanidipine hydrochloride of the present invention is easily incorporated into pharmaceutical compositions and solid dosage forms.

In particular, the invention provides a process for preparing substantially pure amorphous lercanidipine hydrochloride, particularly an amorphous lercanidipine hydrochloride having a purity of at least about 95% and more preferably at least about 97%, even more preferably at least about 99% and still more preferably at least about 99.5%. The purity of the amorphous lercanidipine hydrochloride of the present invention may be determined by any method known in the art, including, but not limited to high performance liquid chromatography (HPLC) analysis.

The process according to the present invention may comprise (a) dissolving lercanidipine free base in an organic solvent to form a first solution, (b) adding to such a first solution a second water and/or organic HCl solution, (c) optionally adding water, and (d) recovering the precipitated amorphous lercanidipine hydrochloride. The temperature is normally in the range from about 10° C to about 60° C, preferably from about 20 to about 40° C.

According to an embodiment, step (a) is carried out by first dissolving lercanidipine free base in an oxygenated organic solvent, selected from (C₁-C₆)-alkanols, preferably methanol and ethanol; (C₃-C₇)- ketones, preferably (C₃-C₆)-ketones, more preferably acetone.

According to a preferred embodiment, I part by weight of lercanidipine free base is dissolved in 1 to 40 volumes of the organic solvent (i.e. g/ml or kg/l); preferably, from 2 to 10 volumes are used when the solvent is an alcohol and/or a ketone.

According to another embodiment, step (b) is carried out by adding under stirring a solution of from 1.0 to 1.5 HCl equivalents, preferably about 1.1 equivalents; the preferred solvent is normally selected from: water; (C₁-C₆)-alkanols, preferably EtOH; (C₂-C₆)-ethers, preferably Et₂O; in particular, water is normally used when the solvent of step (a) is an alcohol and/or a ketone whereas (C₁-C₆)-alknols and/or (C₂-C₆)-ethers are used when the solvent of step (a) is an ether, such as MTBE.

When step (b) is carried out by adding an aqueous HCl solution to an alcohol and/or ketone lercanidipine solution, a viscous solution is normally achieved; in that case, it is advisable to perform step (c) in order to obtain precipitation of the product; this is normally done by adding from 10 to 100 volumes of water, preferably from 20 to 40, with subsequent formation of a gummy solid. On the other hand, if step (b) is carried out by adding an HCl (C₁-C₆)-alkanol and/or (C₂-C₆)-ether solution to an ether lercanidipine solution, a precipitate is directly obtained, thus rendering step (c) useless.

According to an embodiment, the starting product may be lercanidipine hydrochloride in any solid form, i.e. amorphous, crystalline or their admixtures, preferably in crystalline form. Any polymorph or admixture of polymorphs may be used.

The recovery of the thus obtained solid amorphous lercanidipine hydrochloride, i.e. step (d), is achieved with methods known in the art such as ccntrifugation, filtration, and/or drying.

The amorphous lercanidipine hydrochloride obtainable according to the process of the present invention may be used for incorporation into pharmaceutical compositions and solid dosage forms in accordance to the above cited PCT/EP2006/001782, herein incorporated by reverence.

### EXAMPLES

The following examples are illustrative in nature of the various aspects of the present invention and are not intended to be limiting in any manner.

### Example 1A:

1.1 eq of aqueous 1N HCl was added under continuous stirring to a solution of 1 g of lercanidipine free base in 5 mL of acetone. Afterwards, 20 mL of water was added dropwise over a period of 5 min. to the viscous solution obtained. A gummy solid was formed that was isolated by centrifugation, rinsed with water, decanted and dried under vacuum at 30°C (0.15 mmHg) for 4 h affording a dry powder (Yield: 90%). HPLC assay: 99,6%.

### Example 1B:

1.1 eq of aqueous 1N HCl was added under continuous stirring to a solution of 1 g of lercanidipine free base in 5 mL of acetone. The solution obtained after the HCl addition was dropped under stirring into 40 mL of water over a period of 5 min. obtaining a suspension. After 24 hours a gummy solid was formed that was isolated by centrifugation, rinsed with water, filtered, and dried under vacuum (0.15 mmHg) at 30°C for 4 h affording a dry powder (Yield: 85%), HPLC assay: 99,6%.

### Example 2:

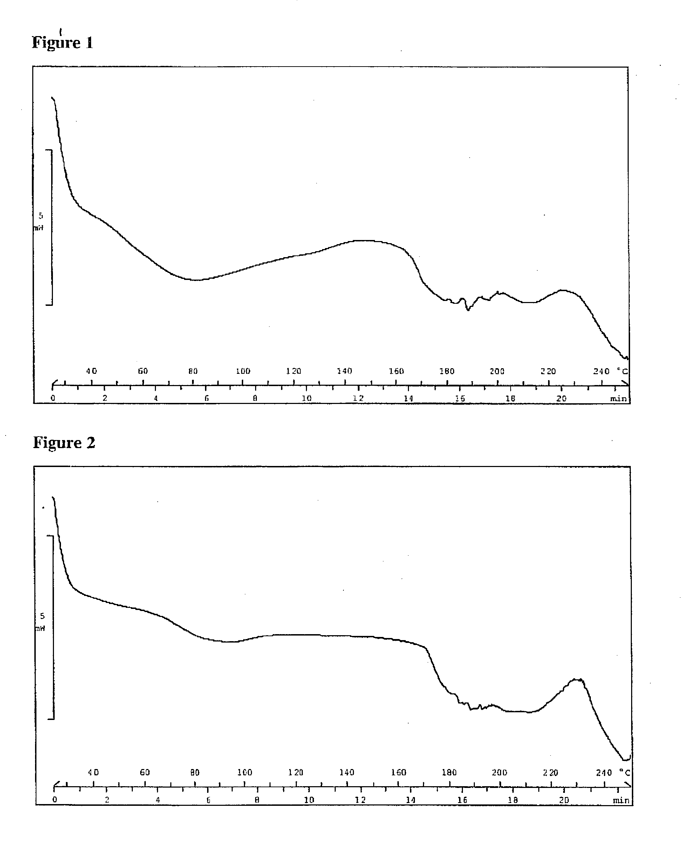

1.1 eq of aqueous 1N HCl was added under continuous stirring to a solution of 1g of lercanidipine free base in 5 mL of EtOH. The solution obtained after the HCl addition was dropped under stirring into 40 mL of water over a period of 5 min. A gummy solid was formed and was isolated by centrifugation, rinsed with water, filtered and dried under vacuum (0.15 mmHg) at 30°C for 4 h affording a dry powder (Yield: 85%), HPLC assay: 99,6%. The DSC of lercanidipine hydrochloride obtained according to the present example is reported in figure 1.

### Example 3A:

1.1 eq. of 2N HCl in EtOH was added dropwise under stirring to a solution of 1 g of lercanidipine free base in 20 mL of methylterbutyl ether (MTBE). The precipitate formed was filtered under vacuum, rinsed with MTBE and dried under vacuum (0.15 mmHg) at 30°C for 4h affording a dry powder (Yield: 90%), HPLC assay: 99,6%.

### Example 3B:

1.1 eq. of 2N HCl in Et₂O was added dropwise under stirring to a solution of 1 g of lercanidipine free base in 20 mL of MTBE. The precipitate formed was filtered under vacuum, rinsed with MTBE and dried under vacuum (0.15 mmHg) at 30°C for 4h affording a dry powder (Yield: 90%), HPLC assay: 99,6%.

### Example 4:

1 g of lercanidipine hydrochloride was dissolved at room temperature in 2 mL of N,N-dimethylformamide. 10 mL of water was added dropwise to the solution, under continuous stirring, over a period of 5 min. The resulting suspension was filtered under vacuum. The solid obtained was rinsed with water and dried under vacuum (0.15 mmHg) at 30 °C for 4 hours affording a dry powder (Yield: 85%), HPLC assay: 99,6%. The DSC of lercanidipine hydrochloride obtained according to the present example is reported in figure 2.

### Example 5:

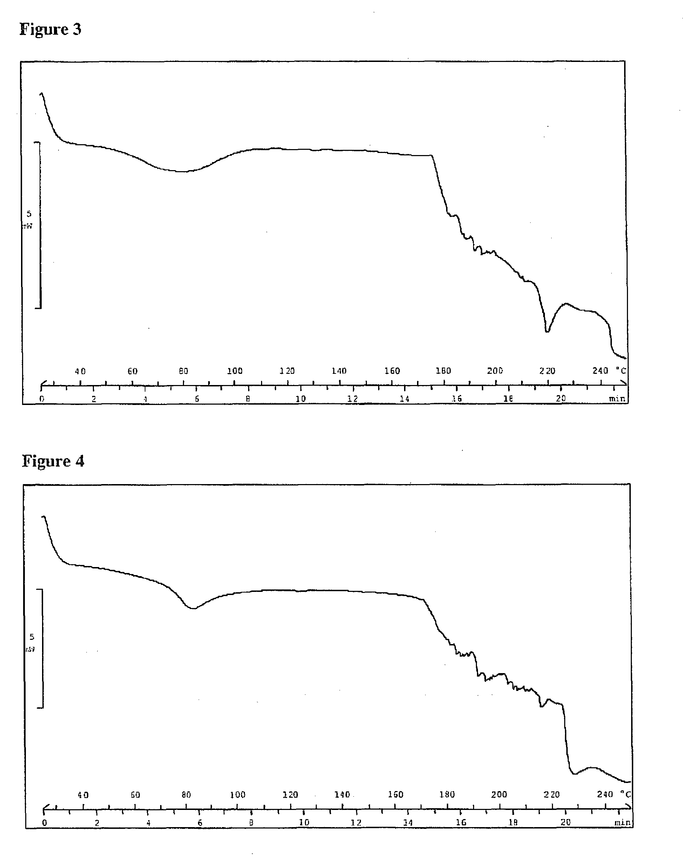

1 g of lercanidipine hydrochloride was dissolved at room temperature in 2 mL of N,N-dimethylformamide. 40 mL of water was quickly added to the solution under vigorous stirring. The mixture was stirred for 24 hours at room temperature. The resulting suspension was filtered under vacuum, the solid washed with water and dried under vacuum (0.15 mmHg) at 50°C for 24 hours affording a dry powder (Yield: 70%). The DSC of lercanidipine hydrochloride obtained according to the present example is reported in figure 3.

### Example 6:

10 g of lercanidipine hydrochloride was dissolved at room temperature in 20 mL of N,N-dimethylformamide. 400 mL of water was quickly added to the solution under vigorous stirring. The mixture was stirred for 24 hours at room temperature. The resulting suspension was filtered under vacuum, the solid washed with water and dried under vacuum (0.15 mmHg) at 50°C for 24 hours affording a dry powder (Yield: 72%). The DSC of lercanidipine hydrochloride obtained according to the present example is reported in figure 4.

### Example 7:

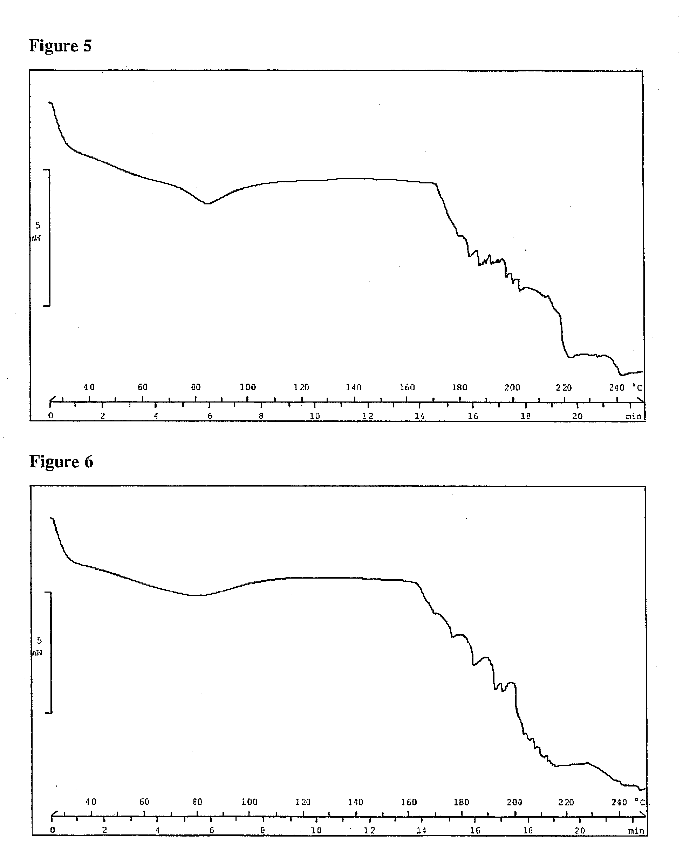

1 g of lercanidipine hydrochloride was dissolved at room temperature in 2 mL of N,N-dimethylacetamide. 40 mL of water was quickly added to the solution under vigorous stirring. The mixture was stirred for 24 hours at room temperature for 24 hours at room temperature. The resulting suspension was filtered under vacuum, the solid washed with water and dried under vacuum (0.15 mmHg) at 50°C for 24 hours affording a dry powder (Yield: 63%). The DSC of lercanidipine hydrochloride obtained according to the present example is reported in figure 5.

### Example 8:

1 g of lercanidipine hydrochloride was dissolved at room temperature in 1.5 mL of N-methylpyrrolidone. 40 mL of water was quickly added to the solution under vigorous stirring. The mixture was stirred for 24 hours at room temperature for 24 hours at room temperature. The resulting suspension was filtered under vacuum, the solid washed with water and dried under vacuum (0.15 mmHg) at 50°C for 24 hours affording a dry powder (Yield: 70%). The DSC of lercanidipine hydrochloride obtained according to the present example is reported in figure 6.

## Claims

1. A process for the manufacture of amorphous lercanidipine hydrochforide which comprises (a) dissolving lercanidipine in an oxygenated organic solvent selected from (C₁-C₆) alkanols and/or (C₃-C₇) ketones to form a first solution, (b) contacting such a first solution with a second water and/or organic HCl solution, (c) optionally adding water and, (d) recovering the precipitated amorphous lercanidipine hydrochloride.

2. A process according to claim 1, **characterized in that** said (C₁-C₆) alkanol is EtOH and/or said (C₃-C₁) ketone is acetone.

3. A process according to claim 1, **characterized in that** the temperature is in the range from about 10 to about 60°C, preferably from about 20 to about 40°C.

4. A process according to claim 1, **characterized in that** step (b) is carried out with a solution of from 1.0 to 1.5 HCl equivalents, preferably about 1.1 equivalents.

5. A process according to claim 4, **characterized in that** the solvent for step (b) is selected from water, (C₁-C₆) alkanols and/or (C₂-C₆) ethers.

6. A process according to claim 5, **characterized in that** said (C₁-C₆) alkalnol is EtOH and/or said (C₂-C₆) ether is Et₂O.

7. A process according to claim 4, **characterized in that** water is used when the solvent of step (a) is an alcohol and/or a ketone.

8. A process according to claim 1, **characterized in that** step (c) is carried out with from 10 to 100 volumes of water, preferably from 20 to 40.

9. A process according to claim 1, **characterized in that** step (d) is carried out by centrifugation, filtration and/or drying.

10. A process according to claim 1, **characterized in that** the obtained amorphous lercanidipine hydrochloride has a purity of at least 95%, preferably of at least 99%, more preferably of at least 99.5%.

11. A process according to claim 1, **characterized in that** the obtained amorphous lercanidipine hydrochloride is substantially free of crystalline lercanidipine hydrochloride.

## Patentansprüche

1. Verfahren zur Herstellung von amorphem
Lercanidipinhydrochlorid, umfassend (a) Auflösen von Lercanidipin in einem sauerstoffhaltigen organischen Lösungsmittel ausgewählt aus (C₁₋₆)-Alkanolen und/oder (C₃₋₇)-Ketonen, um eine erste Lösung zu bilden, (b) in Kontakt bringen einer solchen ersten Lösung mit einer zweiten wässrigen und/oder organischen HCl-Lösung, (c) optionales Hinzufügen von Wasser und (d) Gewinnen des gefällten amorphen Lercanidipinhydrochlorids.

2. Verfahren gemäß Anspruch 1, **dadurch gekennzeichnet, dass** das (C₁₋₆)-Alkanol EtOH ist und/oder das (C₃₋₇)-Keton Aceton ist.

3. Verfahren gemäß Anspruch 1, **dadurch gekennzeichnet, dass** die Temperatur im Bereich von etwa 10 bis etwa 60°C, vorzugsweise von etwa 20 bis etwa 40° C liegt.

4. Verfahren gemäß Anspruch 1, **dadurch gekennzeichnet, dass** Schritt (b) mit einer Lösung von 1,0 bis 1,5 HCl-Äquivalenten, vorzugsweise etwa 1,1 Äquivalenten durchgeführt wird.

5. Verfahren gemäß Anspruch 4, **dadurch gekennzeichnet, dass** das Lösungsmittel für Schritt (b) aus Wasser, (C₁₋₆)-Alkanolen und/oder (C₂₋₆)-Ethern ausgewählt ist.

6. Verfahren gemäß Anspruch 5, **dadurch gekennzeichnet, dass** das (C₁₋₆)-Alkanol EtOH ist und/oder der (C₂₋₆)-Ether Et₂O ist.

7. Verfahren gemäß Anspruch 4, **dadurch gekennzeichnet, dass** Wasser eingesetzt wird, wenn das Lösungsmittel von Schritt (a) ein Alkohol und/oder ein Keton ist.

8. Verfahren gemäß Anspruch 1, **dadurch gekennzeichnet, dass** Schritt (c) mit 10 bis 100 Volumina Wasser, vorzugsweise 20 bis 40, durchgeführt wird.

9. Verfahren gemäß Anspruch 1, **dadurch gekennzeichnet, dass** Schritt (d) durch Zentrifugation, Filtration und/oder Trocknen durchgeführt wird.

10. Verfahren gemäß Anspruch 1, **dadurch gekennzeichnet, dass** das erhaltene amorphe Lercanidipinhydrochlorid eine Reinheit von mindestens 95 %, vorzugsweise mindestens 99 %, stärker bevorzugt mindestens 99,5 %, aufweist.

11. Verfahren gemäß Anspruch 1, **dadurch gekennzeichnet, dass** das erhaltene amorphe Lercanidipinhydrochlorid im wesentlichen frei von kristallinem
Lercanidipinhydrochlorid ist.

## Revendications

1. Procédé pour la fabrication de chlorhydrate de lercanidipine amorphe, qui comprend (a) la dissolution de lercanidipine dans un solvant organique oxygéné choisi parmi les alcanols en C₁ à C₆ et/ou les cétones en C₃ à C₇ pour former une première solution, (b) la mise en contact de cette première solution avec une deuxième solution aqueuse et/ou organique de HCl, (c) éventuellement l'addition d'eau, et (d) la récupération du chlorhydrate de lercanidipine amorphe.

2. Procédé selon la revendication 1, **caractérisé en ce que** ledit alcanol en C₁ à C₆ est l'EtOH et/ou ladite cétone en C₃ à C₇ est l'acétone.

3. Procédé selon la revendication 1, **caractérisé en ce que** la température est située dans la plage allant d'environ 10 à environ 60°C, de préférence d'environ 20 à environ 40°C.

4. Procédé selon la revendication 1, **caractérisé en ce que** l'étape (b) est mise en oeuvre avec une solution de 1,0 à 1,5 équivalents HCl, de préférence d'environ 1,1 équivalents.

5. Procédé selon la revendication 4, **caractérisé en ce que** le solvant pour l'étape (b) est choisi parmi l'eau, les alcanols en C₁ à C₆ et/ou les éthers en C₂ à C₆.

6. Procédé selon la revendication 5, **caractérisé en ce que** ledit alcanol en C₁ à C₆ est l'EtOH et/ ou ledit éther en C₂ à C₆ est l'Et₂O.

7. Procédé selon la revendication 4, **caractérisé en ce que** de l'eau est utilisée quand le solvant de l'étape (a) est un alcool et/ou une cétone.

8. Procédé selon la revendication 1, **caractérisé en ce que** l'étape (c) est mise en oeuvre avec 10 à 100 volumes et de préférence 20 à 40 volumes d'eau.

9. Procédé selon la revendication 1, **caractérisé en ce que** l'étape (d) est mise en oeuvre par centrifugation, filtration et/ou séchage.

10. Procédé selon la revendication 1, **caractérisé en ce que** le chlorhydrate de lercanidipine amorphe obtenu a une pureté d'au moins 95 %, de préférence d'au moins 99 %, mieux encore d'au moins 99,5 %.

11. Procédé selon la revendication 1, **caractérisé en ce que** le chlorhydrate de lercanidipine amorphe obtenu est pratiquement exempt de chlorhydrate de lercanidipine cristallin.
